# EUROPEAN PATENT SPECIFICATION

(11) **EP 2 632 427 B1**
(45) Date of publication and mention of the grant of the patent: **23.03.2016**
(21) Application number: 11778529.5
(22) Date of filing: 28.10.2011
(51) Int. Cl.: A61K 31/5575, A61K 9/00, A61K 47/18, A61K 47/20, A61P 27/02, A61P 27/06

(54) **OPHTHALMIC COMPOSITIONS CONTAINING PROSTAGLANDINS**
PROSTAGLANDINE ENTHALTENDE OPHTHALMISCHE ZUSAMMENSETZUNGEN
COMPOSITION OPHTALMIQUE CONTENANT DES PROSTAGLANDINES

(30) Priority: 05.11.2010 US 410530 P; 29.10.2010 EP 10380137
(43) Date of publication of application: 04.09.2013
(73) Proprietor: Omnivision GmbH, 82178 Puchheim (DE)
(72) Inventor: RIZZO TAMARO, Adriana, E-08191 Rubi (ES)
(74) Representative: Blodig, Wolfgang
(86) International application number: PCT/EP2011/005463
(87) International publication number: WO 2012/055571

(56) References cited:
- EP-A1- 2 127 638
- WO-A2-00/18316
- US-A1- 2010 210 720

## Description

### FIELD OF THE INVENTION

The present invention relates to an aqueous ophthalmic composition containing a prostaglandin such as a prostaglandin FP receptor agonist as active ingredient. The invention also relates to an ophthalmic composition, such as an eye drop formulation, for use in the treatment of glaucoma or intraocular hypertension. The invention further relates to an ophthalmic composition which is free of potentially irritating preservatives such as benzalkonium chloride.

### BACKGROUND OF THE INVENTION

Prostaglandins are well known active ingredients administered to humans or animals via the topical route in the form of ophthalmic solutions for the treatment of glaucoma or intraocular hypertension. The usual dosage for these formulations is 1 drop per day in both eyes. Prostaglandins may also be used in combination with a second anti-glaucoma agent such as a beta-blocker, a carbonic anhydrase inhibitor or an alpha-adrenergic agonist.

The prime disadvantage of prostaglandins is that they have a low water solubility so that special measures have to be taken to stably solubilise the prostaglandin in water. Another constraint on making the formulation is the requirement to provide an ophthalmic solution which is chemically stable over usual storage times. Further, the ophthalmic solution should be stable against adsorbtion of the prostaglandin to the packaging material in which it is stored, in particular relative to packaging in low-density polyethylene (LDPE) material.

As described in US 2010/0210720, currently most prostaglandin based ophthalmic solutions on the market contain a preservative agent which, besides having antimicrobial properties, also solubilises the active ingredient and partly stabilizes it. An example of such a solution is the product marketed under the trade name of Xalatan(R) by Pfizer, containing latanoprost and 0.02% by weight benzalkonium chloride (BAC). Despite the presence of BAC, this ophthalmic solution is not stable at ambient temperature and must be stored in the cold at a temperature of about 5°C.

However, the use of antimicrobial preservatives such as BAC in ophthalmology for long-term treatment is problematic, since it attacks the cornea and causes side-effects such as macular edema and keratoconjunctive disorders. Therefore, it is desired to eliminate preservatives from ophthalmic compositions.

WO 97/29752 discloses the use of a non-ionic agent such as Cremophor™ as partial replacement of BAC. Polysorbate 80 has also been used in ophthalmic solutions as a partial substitute for BAC, as is the case for the product marketed under the trade name Rescula, containing unoprostone with a mixture of BAC and polysorbate 80 forming 0.015% by weight of the solution.

EP 1 321 144 and EP 1 666 043 describe the use of polysorbate 80 to slow down adsorption of a specific prostaglandin, tafluprost, to polyethylene (PE), low density polyethylene (LDPE), polypropylene (PP), polyethylene terephthalate (PET) or a mixtures thereof to the packaging material. These documents state BAC as possible additive for preservation.

US2004/0082660 describes an ophthalmic solution without BAC containing a mixture of latanoprost and polysorbate 80. Experimental data are given on the homogeneity of the solutions in the presence of 0.2 g/l polysorbate 80 thirty minutes after 7 hours of agitation. Thus, this document is concerned with the capacity of polysorbate 80 to solubilise latanoprost in 7 hours.

US 2010/0210720 describes an ophthalmic solution without antimicrobial preservatives. The formulations of the examples include at least one prostaglandin in an amount of 0.005 % and, as a solubilising agent, 0.5 % polyoxyl-15-hydroxystearate (Solutol™ HS15). Thus, the surfactant is used in a huge excess over the active ingredient.

JP 2009-40749-A relates to the problem of providing a stable latanoprost formulation and describes ophthalmic solutions containing the stearic acid ester monostearic acid polyethylene glycol as an inhibitor of adsorption of latanoprost to resin containers. However, the formulations of this document contain BAC for preservation.

Most prostaglandins used in eye drops for the treatment of glaucoma or ocular hypertension are pro-drugs having the prostaglandin carboxylic acid group protected by ester or amide moieties. Use of the pro-drug has the purpose of improving penetration of the compounds into tissue. The active drug which is the free carboxylic acid is formed after application to the eye from the pro-drug by hydrolysis. Among the processes that reduce the amount of the prostaglandin active ingredient in an aqueous solution over time is (apart from adsorbtion to container walls) hydrolysis of the carboxyl-protective group, such as the isopropyl ester group of latanoprost. Although the decomposition product is the substance that acts on the target receptor (the prostaglandin FP receptor), decomposition by hydrolysis in the drug formulation upon storage influences pharmacokinetics, and thus makes the pharmacokinetic properties of the drug time-dependent. Naturally, it is desired that a drug formulation has stable pharmacokinetic properties and concentrations over the usual storage times. In order to account for loss by hydrolysis, excess amounts of pro-drug are sometimes used in order to account for such losses, see EP 1 011 728 B1 of Alcon Manufacturing, Ltd. that uses a 5% excess of the pro-drug in examples 1 and 2. Thus, it is desired to provide stable aqueous prostaglandin formulations wherein hydrolysis can be suppressed during manufacture of the formulations and possibly also during storage.

However, due to the high hydrophobicity of the prostaglandins used in eye drops, preparation of clear aqueous formulations is difficult. In order to dissolve the prostaglandin in the carrier solution, quite harsh conditions such as use of elevated temperatures or use of ultrasound are frequently used. Alternatively, solubilisation takes a long time. For example, heating to 80°C is used in the examples of EP 2 123 278 A1; sonication and stirring overnight is used in US 5,849,792; agitation over 7 hours is done in the example of EP 1 553 953 B1. These methods are, however, disadvantageous from the point of view of avoiding decomposition by hydrolysis. These problems are further reinforced, if established solubilisers such as BAC are to be avoided, and if the content of surfactants that have the potential to influence the surface properties of the cornea is to be reduced as far as possible.

WO 00/18316 A2 describes an ophthalmic composition containing a prostaglandin, a polyanionic polymer and an ion exchange resin. EP2127638 A1 describes use of an anionic surfactant such as polysorbate 80 in the preparation of a preservative-free ophthalmic composition. US 2010/210720 A1 describes a prostaglanin-containing ophthalmic composition without antimicrobial preservatives, containing the anionic surfactant polyoxyl-15-hydroxystearate.

In spite of extensive research over the years, the prior art still does not provide an aqueous ophthalmic composition for the treatment of glaucoma or ocular hypertension that can quickly and stably solubilise prostaglandins using low amounts of surfactants as solubilisers even in the absence of BAC or similar kationic antimicrobial agents and in the presence of low amounts of other surfactants as solubilising agents.

### SUMMARY OF THE INVENTION

Departing from the prior art, it is a problem of the present invention to provide a storage stable ophthalmic composition for use in the treatment of glaucoma or ocular hypertension, wherein the ophthalmic composition is free of antimicrobial agents such as BAC and allows fast and stable solubilisation of prostaglandin anti-glaucoma agents.

Thus, the invention provides an aqueous ophthalmic composition comprising
(i) a prostaglandin; and
(ii) one or more anionic surfactants,
wherein the one or more anionic surfactant is selected from an alkali metal, C₁-C₃ alkylammonium, di(C₁-C₃ alkanol)ammonium, tri(C₁-C₃ alkanol)ammonium, or ammonium salrs of: a C₁₀-C₂₂ alkyl sulfate, a C₁₀-C₂₂ alkyl(oligooxyalkylene)sulfate, wherein the oligooxyalkylene moiety has from one to five oxy-C₁-C₆ alkylene moieties, a C₄-C₂₂ alkylsulfosuccinate ester, a C₁₀-C₂₂ acylsarcosinate, or a C₁₀-C₂₂ alkylcarboxylate;
characterized in that
the concentration of anionic surfactant in the ophthalmic composition is from 0.005 to 0.1 g/L, the concentration of the prostaglandin in the ophthalmic composition is from 0.001 to 0.1 g/ L, and the ophthalmic composition contains less than 0.00005 g/L benzalkonium chloride. In another embodiment, the concentration of the anionic surfactant in the ophthalmic composition is from 0.01 to 0.045 g/L and the concentration of the prostaglandin in the ophthalmic composition is from 0.001 to 0.1 g/L.

The invention also provides a method for manufacture of the aqueous ophthalmic composition of the invention, which comprises the following steps: (i) dissolving a prostaglandin and one or more anionic surfactant in a hydrophilic organic solvent, optionally in the presence of water; and (ii) diluting the solution obtained in step (i) with water optionally including one or more ophthalmically acceptable excipients.

Also provided is a method for manufacture of the aqueous ophthalmic composition of the invention, which comprises the following steps: (i) dissolving a prostaglandin and one or more anionic surfactant in water or in an aqueous solvent; and (ii) diluting the solution obtained in step (i) with water optionally including one or more ophthalmically acceptable excipients.

Further described is a method of preventing or treating glaucoma or intraocular hypertension, or of providing neuroprotection to retinal tissues, comprising topical administration of the aqueous ophthalmic composition of the invention to an affected eye.

The invention further provides a packaged prostaglandin product, comprising the aqueous ophthalmic composition of the invention sealed in a plastic container.

The invention also provides the ophthalmic composition as defined herein for use in the treatment of glaucoma or intraocular hypertension.

The inventor has surprisingly found that anionic surfactants have a high solubilising power for prostaglandin anti-glaucoma agents. As a result, the prostaglandins can be solubilised in water or aqueous solutions in a short period of time even under mild conditions such as cooling to below 10°C or even below 5°C, which suppresses hydrolysis of the prostaglandin pro-drugs. Moreover, storage stable prostaglandin ophthalmic compositions, notably clear solutions thereof for eye drop formulations, containing very small concentrations of the anionic surfactant can be prepared, thus avoiding irritating effects of the surfactant on the eyes. It is even possible to use anionic surfactant concentrations that are below those of the prostaglandin (in terms of weight per volume of formulation). Disclosed herein are prostaglandin eye drop formulations free of BAC or other cationic antimicrobial agents.

### DETAILED DESCRIPTION OF THE INVENTION

The present invention relates to an aqueous ophthalmic composition. The aqueous ophthalmic composition is a clear aqueous solution. The aqueous ophthalmic composition preferably does not comprise particulate matter and is preferably neither a suspension nor a gel.

The aqueous ophthalmic composition of the present invention comprises, in addition to water, a prostaglandin and one or more anionic surfactants as defined in claim 1.

The prostaglandin is preferably an analogue of prostaglandin F_{2α} (Dinoprost). Herein, an analogue of prostaglandin F_{2α} is a compound which comprises a 1,2-disubstituted-3,5-dihydroxycyclopentane ring having the stereochemistry corresponding to prostaglandin F_{2α}. In particular, the analogue of prostaglandin comprises a (1R,2R,3R,5S)-3,5-dihydroxycyclopentane ring having substituents at the 1- position and at the 2-position. Preferably, the 1-position is substituted with a hydrocarbon chain which terminates with a linear or branched C₁-C₆ alkyl ester moiety or a linear or branched C₁-C₆ alkyl amide moiety. The substituent thus may be a C₁-C₆ alkoxycarbonyl-C₆-linear hydrocarbon group or a C₁-C₆ alkylaminocarbonyl-C₆-linear hydrocarbon group. The 2-position of the cyclopentyl ring is substituted with a (CH₂)₂ moiety or a *trans*-CH=CH moiety, to which an optionally fluorinated C₅-C₁₂ hydrocarbon group having at least one hydroxy or oxo substituent or comprising an ether moiety is attached. The

In one embodiment, the prostaglandin is of the formula (1): 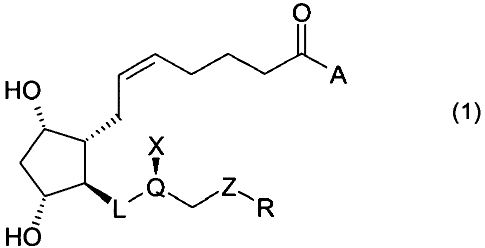 wherein
- A: is a linear or branched C₁-C₆ alkoxy moiety or a linear or branched mono- or di-C₁-C₆ alkylamino moiety;
- L: is a (CH₂)₂ moiety or a *trans*-CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F or H;
- R: is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
- Z: is O or a CH₂ moiety.

In another embodiment, the prostaglandin is of the formula (1), wherein
- A: is a linear or branched C₁-C₆ alkoxy moiety;
- L: is a (CH₂)₂ moiety or a *trans*-CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F;
- R: is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
- Z: is O or a CH₂ moiety.

In a further embodiment, the prostaglandin is of the formula (1), wherein
- A: is a linear or branched C₁-C₆ alkoxy moiety;
- L: is a (CH₂)₂ moiety or a *trans*-CH=CH moiety;
- Q: is C, a CH moiety, or a CF moiety, with the proviso that when
(a) Q is C, X is O and the Q-X bond is a double bond,
(b) Q is CH, X is OH, and
(c) Q is CF, X is F;
- R: is a propyl or pentyl moiety, a phenyl moiety or a 3-(trifluoromethyl)phenyl moiety; and
- Z: is O or a CH2 moiety.

Preferably, A is an isopropoxy moiety or ethylamino moiety, such that the hydrocarbon chain which is the substituent at the 1-position of the prostaglandin (1*R*,2*R*,3*R*,5*S*)-3,5-dihydroxycyclopentane ring terminates with an isopropyl ester moiety or an ethylamide moiety, respectively. More preferably, A is an isopropoxy moiety.

Yet more preferably, the prostaglandin is selected from latanoprost (CAS 130209-82-4), bimatoprost (CAS 155206-00-1), travoprost (CAS 157283-68-6), tafluprost (CAS 209860-87-7) and unoprostone isopropyl (CAS 120373-24-2). Most preferably, the prostaglandin is latanoprost.

The analogue of prostaglandin F_{2α} is preferably a compound which binds to a receptor for prostaglandin F_{2α}. In one embodiment, the prostaglandin is a prostaglandin FP receptor agonist. A prostaglandin FP receptor agonist is preferably a compound which binds to a receptor for prostaglandin F_{2α} and triggers a response therefrom. Such a response preferably involves, *inter alia,* modulation of intraocular pressure. Modulation of intraocular pressure may involve reducing said intraocular pressure through increasing the uveoscleral outflow of the aqueous humor.

The concentration of prostaglandin in the aqueous ophthalmic composition is from 0.001 to 0.1 g/L, preferably from 0.01 to 0.07 g/L, even more preferably from 0.03 to 0.06 g/L. In one embodiment, from 0.045 to 0.55 g/l is used.

The one or more anionic surfactants are surfactants which comprise a hydrophobic organic moiety and a hydrophilic moiety. The hydrophilic moiety is an anion at or near neutral pH values, wherein the anion is selected from sulfate, sulfonate, sarcosinate and carboxylate moieties. The one or more anionic surfactants are selected from C₁₀-C₂₂ alkylsulfates, C₁₀-C₂₂ alkyl(oligooxyalkylene)sulfates, C₄-C₂₂ alkyl sulfosuccinate esters, C₁₀-C₂₂ acylsarcosinatesand C₁₀-C₂₂ alkylcarboxylates; wherein oligooxyalkylene moieties have from one to five oxy-C₁-C₆ alkylene moieties, preferably oxyethylene moieties. Preferably, the upper limit to the alkyl, acyl and fatty acid chain length of C₂₂ in the preceding sentence is C₁₆. Moreover, the hydrocarbon moieties of the alkyl, acyl, alkylene and fatty acid moieties are linear or branched.

The anionic charge on such anionic moieties is countered under aqueous conditions by a countercation. The countercation is selected from alkali metal, C₁-C₃ alkylammonium, tri(C₁-C₃ alkanol)ammonium, di(C₁-C₃ alkanol)ammonium and ammonium cations. In a more preferred embodiment, the countercation is selected from sodium or triethanolammonium cations.

The C₁₀-C₂₂ alkylsulfates, are preferably selected from dodecyl sulfates, tetradecyl sulfates, cocosulfates, tallow alcohol sulfates and mono-C₁₂-C₁₈ alkylsulfates, or mixtures thereof. Preferred examples of such sulfates are selected from sodium dodecylsulfate, potassium dodecylsulfate, triethanolammonium dodecylsulfate (*i.e*. triethanolamine laurylsulfate, CAS 90583-18-9, marketed for example under registered trademark Texapon T42), diethanolammonium dodecylsulfate, ammonium dodecyl sulfate, sodium tetradecyl sulfate, triethanolammonium tetradecyl sulfate, sodium cocosulfates (CAS 97375-27-4), sodium tallow alcohol sulfates (CAS 68140-10-3) and sodium mono-C₁₂-C₁₈ alkylsulfates (CAS 68955-19-1).

The C₁₀-C₂₂ alkyl(oligooxyalkylene)sulfates (also known as alkyl ether sulfates and ether sulfates) are preferably selected from laureth sulfates, trideceth sulfates, myreth sulfates and pentadeceth sulfates, or mixtures thereof such as C₁₂-C₁₄ alkyl(oligooxyalkylene) sulfates (*e.g*. CAS 68891-38-3, marketed under registered trademark Texapon N 40 IS). The laureth sulfates (*e.g*. CAS 3088-31-1, CAS 9004-82-4, CAS 27028-82-6) preferably include ethyleneglycol, diethyleneglycol and triethyleneglycol lauryl ether sulfates, or mixtures thereof. The trideceth sulfates (*e.g*. CAS 25446-78-0) preferably include ethyleneglycol, diethyleneglycol and triethyleneglycol tridecyl ether sulfates, or mixtures thereof. The myreth sulfates (*e.g*. CAS 25446-80-4) preferably include ethyleneglycol, diethyleneglycol and triethyleneglycol myristyl ether sulfates, or mixtures thereof. The pentadeceth sulfates preferably include ethyleneglycol, diethyleneglycol and triethyleneglycol pentadecyl ether sulfates, or mixtures thereof.

The C₄-C₂₂ alkyl sulfosuccinate esters are preferably selected from sulfosuccinate C₁₀-C₂₂ mono-alkyl esters and sulfosuccinate C₄-C₁₂ di-alkyl esters, or mixtures thereof. Preferably, the sulfosuccinate C₁₀-C₂₂ mono-alkyl esters are disodium lauryl sulfosuccinate or disodium myristyl sulfosuccinate. Preferably the sulfosuccinate C₅-C₁₂ dialkyl esters are sodium 1,4-bis(2-ethylhexyl)sulfosuccinate, sodium dioctylsulfosuccinate or sodium 1,4-bis(1-methylheptyl)sulfosuccinate.

The C₁₀-C₂₂ acylsarcosinates (also known as sarcosides) are preferably selected from condensation products of C₁₀-C₂₂ fatty acids, C₁₀-C₂₂ fatty acid halides or C₁₀-C₂₂ fatty acid anhydrides with amino acids or oligopeptides, wherein the oligopeptides may be obtained by partial hydrolysis of collagen. More preferably, the sarcosides are C₁₀-C₁₆ acylsarcosinates selected from sodium lauroyl sarcosinate, potassium cocoyl hydrolysed collagen, triethanolamine cocoyl hydrolysed collagen, potassium undecenoyl hydrolysed collagen and triethanolamine abietoyl hydrolysed collagen.

The C₁₀-C₂₂ alkylcarboxylates (also known as soaps) are preferably selected from decanoates, undecanoates, dodecanoates, tridecanoates, tetradecanoates, pentadecanoates and hexadecanoates, or mixtures thereof.

The one or more anionic surfactants is selected from alkali metal, C₁-C₃ alkylammonium, di(C₁-C₃ alkanol)ammonium, tri(C₁-C₃ alkanol)ammonium, or ammonium salts of C₁₀-C₂₂ alkylsulfates, C₁₀-C₂₂ alkyl(oligooxyalkylene)sulfates, C₄-C₂₂ alkyl sulfosuccinate esters, C₁₀-C₂₂ acylsarcosinates and C₁₀-C₂₂ alkylcarboxylates, wherein oligooxyalkylene moieties represent from one to five oxy-C₁-C₆ alkylene moieties, preferably oxyethylene moieties, linked via ether moieties.

In a further preferred embodiment, the one or more anionic surfactants are selected from alkali metal, C₁-C₃ alkylammonium, di(C₁-C₃ alkanol)ammonium, tri(C₁-C₃ alkanol)ammonium, or ammonium salts of a C₁₀-C₂₂ alkyl sulfate, a C₁₀-C₂₂ alkyl(oligooxyalkylene)sulfate, a C₄-C₂₂ alkyl sulfosuccinate ester and a C₁₀-C₂₂ acylsarcosinate, and
(i) a C₁₀-C₂₂ alkyl sulfate is more preferably selected from sodium dodecylsulfate (CAS 151-21-3), potassium dodecylsulfate (CAS 4706-78-9), triethanolammonium dodecylsulfate (CAS 90583-18-9), diethanolammonium dodecylsulfate (CAS 143-00-0), ammonium dodecyl sulfate (CAS 2235-54-3), sodium tetradecyl sulfate (CAS 1191-50-0), triethanolammonium tetradecyl sulfates (CAS 4492-78-8), sodium cocosulfates (CAS 97375-27-4), sodium tallow alcohol sulfates (CAS 68140-10-3), triethanolammonium C₁₂-C₁₄ alkylsulfates (CAS 90583-18-9) and sodium C₁₂-C₁₈ alkylsulfates (CAS 68955-19-1);
(ii) a C₁₀-C₂₂ alkyl(oligooxyalkylene)sulfate is more preferably selected from sodium laureth sulfate (CAS 3088-31-1, CAS 9004-82-4), triethanolamine laureth sulfate (CAS 27028-82-6), sodium trideceth sulfate (CAS 25446-78-0), sodium myreth sulfate (CAS 25446-80-4) and sodium C₁₂-C₁₄ alkyl(oligooxyethylene) sulfates (CAS 68891-38-3);
(iii) a C₄-C₂₂ alkyl sulfosuccinate ester is more preferably selected from disodium lauryl sulfosuccinate (CAS 13192-12-6), sodium 1,4-bis(2-ethylhexyl)sulfosuccinate (CAS 577-11-7), sodium dioctylsulfosuccinate (CAS 2373-23-1), sodium dicyclohexylsulfosuccinate (CAS 23386-52-9) and sodium 1,4-bis(1-methylheptyl)sulfosuccinate (CAS 20727-33-7); and (iv) a C₁₀-C₂₂ acylsarcosinate is more preferably selected from sodium lauroyl sarcosinate (CAS 137-16-6), potassium cocoyl hydrolysed collagen (CAS 68920-65-0), triethanolamine cocoyl hydrolysed collagen (CAS 68952-16-9), potassium undecenoyl hydrolysed collagen (CAS 68951-92-8) and triethanolamine abietoyl hydrolysed collagen (CAS 68918-77-4).

The concentration of anionic surfactant in the ophthalmic composition is from 0.005 to 0.1 g/L, preferably 0.005 to 0.05 g/L and the ophthalmic composition contains less than 0.00005 g/L benzalkonium chloride.

In a further embodiment, the concentration of anionic surfactant is from 0.01 to 0.045 g/L. The term "concentration of anionic surfactant" means the sum of concentrations of anionic surfactants if more than one anionic surfactant is used. However, it is preferred that only one type of anionic surfactant is used in the composition to keep the mixture simple.

In one embodiment, the ratio of the weight of the prostaglandin to the total weight of anionic surfactant(s) [*i.e*. the weight ratio of the prostaglandin to anionic surfactant(s)] in the aqueous ophthalmic composition is 1.0 or more. In a preferred embodiment, the weight ratio of the prostaglandin to anionic surfactant(s) is from 1.0 to 20.0, more preferably from 2.0 to 20.0. Note that ratios reported herein are expressed as the quotient of the ratio (*e.g*. a ratio ranging from 6:2 to 6:1 is expressed as 3.0 to 6.0).

The ophthalmic composition contains less than 0.00005 g/ L benzalkonium chloride preferably at most 0.00001 g/L or less. It is preferred that the aqueous ophthalmic composition comprises no cationic surfactants. When the amount of benzalkonium chloride or other cationic surfactants in the aqueous ophthalmic composition is greater than the above upper limit, irritating effects on the cornea may occur, and unfavourable interactions with the anionic surfactant used in the invention may occur. "Other cationic surfactants" that are not included beyond the upper limits given above are polymeric quaternary ammonium compounds such as those given in paragraph [0052] of US 2009/0234013. The wording "total at most" used above means that the total amount of any benzalkonium chloride plus the amount of any other cationic surfactant together is at most the upper limits given above.

In one embodiment, the aqueous ophthalmic composition additionally comprises a hydrophilic organic solvent. The hydrophilic organic solvent is an organic solvent that exhibits a solubulity in water of at least 10 g/L, preferably at least 20 g/L, more preferably at least 50 g/L at 25°C. Most preferably the hydrophilic organic solvent is completely miscible in water. Preferably, the hydrophilic organic solvent is selected from one or more linear, branched or cyclic C₁-C₆ alcohols, polyols or ketones, acetonitrile, dimethylsulfoxide, dimethylformamide, and tetrahydrofuran. In a preferred embodiment the hydrophilic organic solvent is a polyol, more preferably a C₁-C₆ alkylene glycol, a poly-C₂-C₆ alkylene glycol or a C₁-C₆ alkane triol. Preferably, the hydrophilic organic solvent is selected from ethylene glycol, 1,2-propane diol, 1,3-propane diol, glycerol, sugar alcohols such as erythritol, arabitol, mannitol, sorbitol, xylitol, polyethylene glycols such as PEG 200, PEG 400, PEG 1000, polypropyleneglycols such as PPG 200, PPG 1000, and copolymers of polyethylene glycol and polypropylene glycol such as the block copolymer Synperonic™ PE/L61. Such hydrophilic organic solvents may be used singly or in combination. More preferably, the hydrophilic organic solvent is selected from at least one of ethylene glycol, 1,2-propane diol, 1,3-propane diol and glycerol.

The ratio of the total volume of hydrophilic organic solvent to total volume of water which is present in the aqueous ophthalmic composition is preferably from 0.01 or less. More preferably, the volume ratio of organic solvent to water in the ophthalmic composition is from 0.005 to 0.0001, most preferably from 0.002 to 0.0001. These volume ratios refer to amounts before mixing the hydrophilic organic solvent and water.

The aqueous ophthalmic composition of the present invention may be a buffered aqueous solution. Preferably, the aqueous ophthalmic composition is buffered so as to have a pH which is within 0.5 pH units (when measured using universal indicator paper) of the pH of tears. Preferably, the pH of the ophthalmic composition is from 6.4 to 7.0, more preferably from 6.6 to 6.8. Preferably, the buffer system deployed in the buffered aqueous solution is a phosphate buffer, more preferably a K₂HPO₄/KH₂PO₄ buffer or a Na₂HPO₄/NaH₂PO₄ buffer.

The aqueous ophthalmic composition of the present invention is preferably isoosmotic and isotonic with the fluids found in the ocular cavity, namely tears. Preferably, the osmolarity of the aqueous ophthalmic composition is from 260-315 mOsm/L at 25°C. Thus, the aqueous ophthalmic composition preferably comprises one or more salts, preferably sodium chloride. The amount of such salts comprised in the aqueous ophthalmic composition is adjusted to provide a composition with an osmolarity and/or tonicity which is isoosmotic and/or isotonic with the fluids found in the ocular cavity. Any suitable solutes may be used for setting the osmolarity of the ophthalmic composition. Mannitol is an example of a suitable osmotic agent that can be added in an amount to achieve the desired osmolarity or isoosmotic conditions.

The aqueous ophthalmic composition of the present invention may additionally comprise one or more excipients. Preferably, such excipients are excipients which are acceptable for ophthalmic administration in that they are compatible with the tissues and fluids found in the ocular cavity. The excipients may be selected from non-ionic surfactants, antimicrobial agents, hydrophilic polymers and antioxidants.

Non-ionic surfactants are selected from ethoxylates, fatty acid esters of polyols and amine oxides. Ethoxylates (*e.g*. polyoxyl 40 stearate, Myrj 52, Brij 30, Tween 20, Tween 40, Tween 60, Tween 65, Tween 80, polyoxyl 40 hydrogenated castor oil) are preferably selected from ethoxylated, diethoxylated, triethoxylated, tetraethoxylated, pentaethoxylated or hexaethoxylated C₁₀-C₂₂ fatty acids, C₁₀-C₂₂ fatty acid amides, C₁₀-C₂₂ fatty amides, C₁₀-C₂₂ alcohols, C₁₀-C₂₂ alkylphenols and C₁₀-C₂₂ fatty acid esters of polyols, wherein the hydrogen atom of the terminal hydroxyl group is optionally replaced with a hydrophobic moiety such as a benzyl, butyl or methyl group, or mixtures thereof. Fatty acid esters of polyols (*e.g*. sorbityl monolaurate, sorbityl monooleate, CAS 68308-54-3, CAS 128664-36-8, CAS 136797-44-9) are preferably selected from C₁₀-C₂₂ fatty acid esters of glycerol, sorbitol, sucrose and polyglucosides, or mixtures thereof. Amine oxides (*e.g*. CAS 1643-20-5, CAS 2571-88-2, CAS 61788-90-7) are preferably selected from C₁₀-C₂₂ alkyldimethylamine oxides and C₁₀-C₂₂ alkyldiethanolamine oxides. The total amounts of non-ionic surfactants in the ophthalmic composition should be lower than 5.0 g/L, preferably lower than 1.0 g/L. It may also be lower than 0.1 g/L. In one embodiment, the amount of non-ionic surfactant is lower than the amount of the anionic surfactant present in the ophthalmic composition in terms of mass per volume of the composition.

Anti-microbial agents may be added to the aqueous ophthalmic composition in order to prevent microbes from growing and/or reproducing in the aqueous ophthalmic composition. The anti-microbial agents are selected from antibiotics and antifungals. Preferably the anti-microbial agent is selected from ethylene oxide, boric acid, carbapenems, cephalosporins, penicillins, quinolones, sulfonamides, tetracyclines, polyene, imidazole, triazole, thiazole, allylamines and echinocandins. The anti-microbial agent can be added in an amount sufficient to halt and/or inhibit microbial growth in the aqueous ophthalmic composition for the duration of its storage prior to expiry. When the aqueous ophthalmic composition of the present invention is packaged in a single dose package, ethylene oxide may be used to sterilize the package prior to sealing.

Hydrophilic polymers may be added to the aqueous ophthalmic composition in order to thicken the tear film and increase corneal contact time of the prostaglandin. Preferably, the hydrophilic polymers are selected from methylcellulose, hydroxyethylcellulose, hydroxypropylmethylcellulose, polyethylene glycol, polyvinylpyrrolidone, dextran and polyvinylalcohol. The hydrophilic polymers may be used in an amount sufficient to provide the aqueous ophthalmic composition with a viscosity in the range of 5 to 100 cps at 20 °C.

Antioxidants may be added to the aqueous ophthalmic composition in order to prevent oxidation of the prostaglandin. Examples of antioxidants are ascorbic acid, acetylcysteine, EDTA and sodium bisulfite.

The ophthalmic composition of the present invention may comprise a further active ingredient in addition to the prostaglandin. For example, the ophthalmic composition may contain one or more further active agent for the treatment of glaucoma or intraocular hypertension. A compound class commonly used for the treatment of glaucoma or intraocular hypertension are β-blockers. A preferred β-blocker is timolol or a salt thereof such as timolol maleate.

The present invention also relates to a method for manufacture of the herein-described aqueous ophthalmic composition. The method for manufacture of the aqueous ophthalmic composition comprises dissolving the ingredients in water in a one step or multi-step procedure. Disclosed herein are methods which comprise dissolving the prostaglandin in the presence of the one or more anionic surfactant in a one step or multi-step procedure. In a preferred embodiment, the procedure has two or more steps.

The two-step method comprises a first step of dissolving the prostaglandin and the one or more anionic surfactants in water or, preferably, in a hydrophilic organic solvent or water/ hydrophilic organic solvent mixture. Thus, the first step of dissolution is performed either in the absence of water or in the presence of water. When the step of dissolution is performed in the presence of water, water may be added to the hydrophilic organic solvent as pure water, as an aqueous solution comprising the one or more anionic surfactants, as an aqueous saline solution or as an aqueous buffer solution. It is preferred to include the entire amount of the prostaglandin and the entire amount of the anionic surfactant to be included in the ophthalmic composition in the first step. Dissolution is preferably performed by agitation of the mixture of a prostaglandin and one or more anionic surfactants in a hydrophilic organic solvent, optionally in the presence of water. Agitation may be by shaking, swirling or stirring.

The two-step method additionally comprises a second step of diluting the solution obtained in the first step with water optionally including one or more ophthalmically acceptable excipients. Thus, the second step of dissolution is performed by adding water to the solution obtained in the first step, or by adding an aqueous solution comprising one or more ophthalmically acceptable excipients in the presence of water. Preferably an aqueous solution comprising one or more ophthalmically acceptable excipients is an aqueous buffer solution or an aqueous saline solution. In the second step, dilution with water of the solution obtained in the first step may be made in one or more sub-steps by adding the water in two or more portions.

The method of the present invention may additionally comprise further steps of sterilization and/or packaging.

The step of sterilizing is selected from a step of filtration with a microbial filter or an ultrafilter, and/or chemical sterilization by treatment with an antimicrobial agent or by displacement of oxygen from the solution. When the method for manufacture of the aqueous ophthalmic composition comprises a step of filtration, this is preferably employed as a third step, wherein the diluted solution obtained in the second step is subjected to filtration. As filtration device, the Milliflex™ system used in the examples may be employed. For larger volumes, SartobranP™ from Sartorius may be employed. When the method for manufacture of the aqueous ophthalmic composition comprises a step of chemical sterilization, this is effected before, during or after any of the first and second steps.

The step of packaging involves transferring the aqueous ophthalmic composition of the present invention to one or more packages. Each package is a sealable package which may be a single-dose or multiple-dose package. The package preferably comprises plastic and/or glass containers, wherein the plastic is a plastic selected from polyethylene, polypropylene and/or a copolymer of polyethylene and polypropylene, more preferably polyethylene. The package is selected from a one-piece package or a two-piece package comprising a base and a cap. Optionally, the package may comprise a valve, dropper, or plunger for dispensing the aqueous ophthalmic composition of the present invention. The step of packaging preferably deploys blow-fill-seal technology, e.g. to form single-dose packages comprising the aqueous ophthalmic composition of the present invention. Alternatively, the step of packaging preferably involves transferring the aqueous ophthalmic composition of the present invention to one or more multiple-dose packages. When the method of the present invention involves a step of packaging in a multiple-dose package, the aqueous ophthalmic composition of the present invention preferably comprises an antimicrobial agent. Multiple-dose packages may contain a filter for preventing microbial contamination as described in EP 2 193 795 A1.

Single-dose packages may contain between 0.2 and 0.8 ml, preferably between 0.3 to 0.5 ml per package. Multi-dose packages may contain between 1 and 25 ml, preferably between 2 and 10 ml per package.

Preferably, the method for manufacture of the aqueous ophthalmic composition of the present invention is performed at between 0 and 10°C, more preferably between 3 and 8°C, most preferably at between 5 and 7°C. In one embodiment, either or both of the two steps (or any further step) of the method for manufacture of the aqueous ophthalmic composition of the present invention are performed at between 0 and 10 °C, preferably between 3 and 8 °C. Preferably, both of the two steps of the method of manufacture are performed at between 0 and 10 °C, more preferably between 3 and 8 °C. When the method for manufacture of the aqueous ophthalmic composition of the present invention is performed at temperature as defined above, degradation of the prostaglandin can be suppressed.

Preferably, the method for manufacture of the aqueous ophthalmic composition of the present invention is also performed in the absence of oxygen under a non-oxidising atmosphere, more preferably under a nitrogen or argon atmosphere. When the method of the present invention comprises a step of packaging, this step is preferably performed under a nitrogen atmosphere.

The present invention may be used for preventing or treating glaucoma or intraocular hypertension, or of providing neuroprotection to retinal tissues, comprising topical administration of the aqueous ophthalmic composition of the present invention. Topical administration of the aqueous ophthalmic composition is effected by applying said ophthalmic composition to the ocular cavity, preferably by administration to at least one of the conjunctiva, cornea, sclera and the inner surfaces of the eyelid.

Preferably, the aqueous ophthalmic composition is administered in doses of a volume and frequency sufficient to reduce the intraocular pressure. Preferably, one or a few drops are applied to the ocular cavity with each administration. Preferably the aqueous ophthalmic composition of the present invention is administered in such doses from 1 to 6 times daily, more preferably from 1 to 3 times daily.

### EXAMPLES

### Example 1

A latanoprost formulation of batch size 7.5 liters was prepared from the following components:

| Ingredient | Quantity |
|---|---|
| latanoprost | 0.375 g |
| disodium phosphate | 35.55 g |
| monosodium phosphate monohydrate | 34.5 g |
| TEXAPON™ N 40 IS | 0.55 g |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

TEXAPON™ N 40 IS was obtained from Cognis. It is a solution containing 27% sodium lauryl ether sulfate. The propylene glycol used in this and the following examples is propane-1,2-diol.

First, a latanoprost solution was made as follows. Latanoprost, sodium lauryl ether sulfate and propylene glycol were mixed in the amounts indicated in the above table in a glass vessel. The vessel was shaken gently while cooling on a water/ice mixture until the components dissolved, which takes about 5 minutes. Then, 5 ml of water were added and shaken gently for another 5 minutes, while cooling was continued. Then, 10 ml of water were added and shaken gently for 10 minutes during which time cooling was continued.

In a separate vessel, the disodium phosphate, the monosodium phosphate monohydrate and the sodium chloride was dissolved in about 6 I of water (80% of the total volume of the batch size). When the solids were dissolved, the latanoprost solution made as described in the preceding paragraph was added, followed by gentle shaking for 10 minutes. Then, the pH was set to be within the range of 6.6-6.8 with the 1.0 M sodium hydroxide solution or the diluted phosphoric acid as necessary. Next, water was added up to the final volume of 7.5 I.

The obtained mixture was filtered through a Milliflex™-100 equipment using a 0.2 µm membrane. The filtrate was dispensed into 2.5 ml PE bottles in a bottlepack™ system, and used in the tests of the following example 2. The bottlepack system allows filling of the bottles under sterile conditions under laminar air flow. The bottles were filled using the blow-fill-seal methodology under nitrogen as an inert gas. A Rommelag™ bottlepack™ 31226187 machine was used for this purpose.

### Example 2

The latanoprost formulation of Example 1 was tested on its content of latanoprost and degradation products of latanoprost:
- before and after the filtration through the 0.2 µm membrane of Example 1;
- in a short stability test.
The Milliflex™-100 filter system mentioned above was used for filtration. The microbiology test was made according to the European Pharmacopeia. In the appearance test, any change in the solution such as color or turbidity was checked visually. The concentration of the latanoprost in the solutions was measured by HPLC and determined by internal standard method with a calibration curve using a Kromasil™ C18 column. For the degradation products, a Kromasil™ 10 CelluCoat™ column was used.

The results of 3 independent laboratory batches before and after filtration are as follows:

| Batch LTX-01 | | |
|---|---|---|
| Parameter | Before filtration | After filtration |
| Appearance | Complies | Complies |
| pH | 6.69 | 6.62 |
| Latanoprost assay (µg/ml) | 50.33 | 50.19 |
| Sum of all degradation products | 0.058% | 0.063% |
| Microbiological control | < 10 CFU/100 ml | sterile |
| | | |

| Batch LTX-02 | | |
|---|---|---|
| Parameter | Before filtration | After filtration |
| Appearance | Complies | Complies |
| pH | 6.72 | 6.72 |
| Latanoprost assay (µg/ml) | 49.96 | 49.88 |
| Sum of all degradation products | 0.071 % | 0.073% |
| Microbiological control | < 10 CFU/100 ml | sterile |
| | | |

| Batch LTX-03 | | |
|---|---|---|
| Parameter | Before filtration | After filtration |
| Appearance | Complies | Complies |
| pH | 6.78 | 6.78 |
| Latanoprost assay (µg/ml) | 50.04 | 49.89 |
| Sum of all degradation products | 0.053% | 0.051% |
| Microbiological control | < 10 CFU/100 ml | sterile |

The above data show that essentially no latanoprost was lost in the filtration step.

The stability test was conducted on 3 separate batches of formulations. The formulations were stored at a temperature of 5-8°C for 30 days at a relative humidity of 60% in sterilised 50 ml glass bottles. The results from 3 separate tests are as follows.

| Batch LTX-01 | | |
|---|---|---|
| Parameter | Time 0 | Time 30 days |
| Appearance | Complies | Complies |
| pH | 6.62 | 6.63 |
| Latanoprost assay (µg/ml) | 50.19 | 50.18 |
| Sum of all degradation products | 0.063% | 0.061% |
| Microbiological control | < 10 CFU/100 ml | sterile |
| | | |

| Batch LTX-02 | | |
|---|---|---|
| Parameter | Time 0 | Time 30 days |
| Appearance | Complies | Complies |
| pH | 6.72 | 6.73 |
| Latanoprost assay (µg/ml) | 49.88 | 49.79 |
| Sum of all degradation products | 0.073% | 0.069% |
| Microbiological control | < 10 CFU/100 ml | Sterile |
| | | |

| Batch LTX-03 | | |
|---|---|---|
| Parameter | Time 0 | Time 30 days |
| Appearance | Complies | Complies |
| pH | 6.78 | 6.77 |
| Latanoprost assay (µg/ml) | 49.89 | 49.92 |
| Sum of all degradation products | 0.051% | 0.053% |
| Microbiological control | < 10 CFU/100 ml | Sterile |

The data from the above tables demonstrate that there was no noticeable loss of latanoprost under the conditions used.

### Example 3

Latanoprost formulations with alternative anionic surfactants. Batch size: 7.5 liters.

| Ingredients | Quantity |
|---|---|
| latanoprost | 0.375 g |
| disodium phosphate | 35.55 g |
| monosodium phosphate monohydrate | 34.5 g |
| | |
| anionic surfactant: | 0.150 g |
| sodium lauryl ether sulfate or | |
| sodium lauroyl sarcosinate or | |
| triethanolamine lauryl ether sulfate | |
| | |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

For each of the 3 anionic surfactants listed in the above table, separate latanoprost formulations were made following the procedure described in Example 1 using the quantities of components given in the above table. Triethanolamine lauryl sulfate was the commercial product TEXAPON™ T42. The amounts given for the surfactants are those of the indicated chemical substance. The 3 formulations can be tested as described in Example 2.

| Batch LTX 05 - Lauryl ether sulfat sodium | |
|---|---|
| Parameter | Time 0 |
| Appearance | Complies |
| pH | 6.64 |
| Latanoprost assay (µg/ml) | 50.44 |
| Sum of all degradation products | 0.015% |
| Microbiological control | < 10 CFU/100 ml |

| Batch LTX-06 - Lauroyl sarcosinate sodium | |
|---|---|
| Parameter | Time 0 |
| Appearance | Complies |
| pH | 6.69 |
| Latanoprost assay (µg/ml) | 50.14 |
| Sum of all degradation products | 0.019% |
| Microbiological control | < 10 CFU/100 ml |
| | |

| Batch LTX-07 - Lauryl ether triethanolamine | |
|---|---|
| Parameter | Time 0 |
| Appearance | Complies |
| pH | 6.70 |
| Latanoprost assay (µg/ml) | 50.12 |
| Sum of all degradation products | 0.018% |
| Microbiological control | < 10 CFU/100 ml |

### Example 4

Latanoprost formulations containing different concentrations of anionic surfactants. Formulation for batch size of 7.5 liters.

| Ingredients | Quantity |
|---|---|
| latanoprost | 0.375 g |
| disodium phosphate | 35.55 g |
| monosodium phosphate monohydrate | 34.5 g |
| | |
| anionic surfactant: | |
| sodium lauryl ether sulfate | 0.150 g |
| or | 0.300 g |
| or | 0.450 g |
| | |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

For each of the 3 anionic surfactant concentrations of sodium lauryl ether sulfate listed in the above table, a separate latanoprost formulation was made following the procedure described in Example 1. The 3 formulations can be tested as described in Example 2.

The times required for dissolution in the first dissolution step wherein latanoprost, the sodium lauryl ether sulfate and propylene glycol are mixed in a glass vessel as described in Example 1 were measured:

| Batch | Time for dissolution of latanoprost |
|---|---|
| Batch LTX 08 with 0.150 g surfactant | < 5 minutes |
| Batch LTX 09 with 0.300 g surfactant | < 5 minutes |
| Batch LTX 010 with 0.450 g surfactant | < 5 minutes |

| Batch LTX 08 - Lauryl ether sulfat sodium 150 mg | |
|---|---|
| Parameter | Time 0 |
| Appearance | Complies |
| pH | 6.72 |
| Latanoprost assay (µg/ml) | 50.31 |
| Sum of all degradation products | 0.019% |
| Microbiological control | < 10 CFU/100 ml |
| | |

| Parameter | Time 0 |
|---|---|
| Appearance | Complies |
| pH | 6.72 |
| Latanoprost assay (µg/ml) | 50.26 |
| Sum of all degradation products | 0.021% |
| Microbiological control | < 10 CFU/100 ml |
| | |

| Parameter | Time 0 |
|---|---|
| Appearance | Complies |
| pH | 6.69 |
| Latanoprost assay (µg/ml) | 50.28 |
| Sum of all degradation products | 0.015% |
| Microbiological control | < 10 CFU/100 ml |

### Example 5

Comparative tests on the speed of dissolution of latanoprost with different surfactants. Formulation for batch size of 7.5 liters.

| Ingredients | Quantity |
|---|---|
| latanoprost | 0.375 g |
| disodium phosphate | 35.55 g |
| monosodium phosphate monohydrate | 34.5 g |
| | |
| surfactant: | 0.300 g |
| sodium lauryl ether sulfate | |
| or Cremophor RH40 | |
| or Polysorbate 80 | |
| | |
| propylene glycol | 5 ml |
| sodium chloride | 30.75 g |
| 1.0 M NaOH or diluted phosphoric acid | as required for final pH 6.6-6.8 |
| water for injection | for final volume of 7.5 l |

For each of the 3 surfactants listed in the above table, a separate latanoprost formulation was made using the same amount of the surfactants indicated in the above table following the procedure described in Example 1.

The times required for dissolution in the first dissolution step wherein latanoprost, the surfactant and propylene glycol are mixed in a glass vessel as described in Example 1 were measured. The results are as follows:

| Batch | Time for dissolution of latanoprost |
|---|---|
| Batch LTX 011 with sodium lauryl ether sulfate | < 5 minutes |
| Batch LTX 12 with Cremophor RH40 | 23 minutes |
| Batch LTX 013 with Polysorbate 80 | 37 minutes |

At 0°C, Cremophor RH40 and Polysorbate 80 are not liquid. The presence of a creamy substance was observed until all the material was dissolved.

### Example 6

A travoprost formulation of bach size 7.5 litres was prepared from the following components:

| Ingredient | Quantity |
|---|---|
| travoprost | 0.300 g |
| boric acid | 30.75 g |
| trometamol | 4.95 g |
| sodium lauryl ether sulfate 27% | 0.55 g* |
| manitol | 34.50 g |
| 1.0 M NaOH or diluted HCl | as required for final pH 5.8-6.2 |
| water for injection | for final volume of 7.5 l |

| | |
|---|---|
| * of the 27% solution | |

First, a travoprost solution was made as follows. Travoprost, sodium lauryl ether sulfate and 20 ml of water were mixed in the amounts indicated in the above table in a glass vessel. The vessel was sonicated at room temperature until the components dissolved, which takes about 5 minutes. Then, 300 ml of water were added and sonicated for another 5 minutes. The temperature of the solution does not rise above 25°C.

In a separate vessel, the boric acid, the trometamol and the manitol was dissolved in about 6 I of water (80% of the total volume of the batch size). When the solids were dissolved, the travoprost solution made as described in the preceding paragraph was added, followed by gentle shaking for 10 minutes. Then, the pH was set to be within the range of 5.8-6.2 with the 1.0 M sodium hydroxide solution or the diluted hydrochloric acid as necessary. Next, water was added up to the final volume of 7.5 I.

The obtained mixture was filtered through a Millipore filter using a 0.2 µm membrane. The filtrate was dispensed into 2.5 ml PE bottles in a bottlepack system, and used in the tests of the following example 7.

### Example 7

The travoprost formulation of Example 6 was tested on its content of travoprost and degradation products of travoprost:
- before and after the filtration through the 0.2 µm membrane of Example 6
- in a short stability test.

The results of 3 independent laboratory batches before and after filtration are as follows:

| Batch TVX-01 | | |
|---|---|---|
| Parameter | Before filtration | After filtration |
| Appearance | Complies | Complies |
| pH | 6.11 | 6.11 |
| Travoprost assay (µg/ml) | 41.2 | 41.3 |
| Sum of all degradation products | < 0.05% | < 0.05% |
| Microbiological control | < 10 CFU/100 ml | sterile |
| | | |

| Batch TVX-02 | | |
|---|---|---|
| Parameter | Before filtration | After filtration |
| Appearance | Complies | Complies |
| pH | 5.94 | 5.92 |
| Travoprost assay (µg/ml) | 40.7 | 40.5 |
| Sum of all degradation products | < 0.05% | < 0.05% |
| Microbiological control | < 10 CFU/100 ml | sterile |
| | | |

| Batch TVX-03 | | |
|---|---|---|
| Parameter | Before filtration | After filtration |
| Appearance | Complies | Complies |
| pH | 6.03 | 6.03 |
| Travoprost assay (µg/ml) | 40.16 | 40.19 |
| Sum of all degradation products | < 0.05% | < 0.05% |
| Microbiological control | < 10 CFU/100 ml | sterile |

The above data show that essentially no travoprost was lost in the filtration step.

The stability test was conducted on 3 separate batches of formulations. The formulations were stored at a temperature of 5-8°C for 30 days at a relative humidity of 60%.

| Batch TVX-01 | | |
|---|---|---|
| Parameter | Time 0 | Time 30 days |
| Appearance | Complies | Complies |
| pH | 6.11 | 6.13 |
| Travoprost assay (µg/ml) | 41.3 | 41.2 |
| Sum of all degradation products | < 0.05% | < 0.05% |
| Microbiological control | sterile | sterile |
| | | |

| Batch TVX-02 | | |
|---|---|---|
| Parameter | Time 0 | Time 30 days |
| Appearance | Complies | Complies |
| pH | 5.92 | 5.91 |
| Travoprost assay (µg/ml) | 40.5 | 40.2 |
| Sum of all degradation products | < 0.05% | < 0.05% |
| Microbiological control | sterile | sterile |
| | | |

| Batch TVX-03 | | |
|---|---|---|
| Parameter | Time 0 | Time 30 days |
| Appearance | Complies | Complies |
| pH | 6.03 | 6.03 |
| Travoprost assay (µg/ml) | 40.19 | 40.20 |
| Sum of all degradation products | < 0.05% | < 0.05% |
| Microbiological control | sterile | sterile |

The data from the above tables demonstrate that there was no noticeable loss of travoprost under the conditions used.

### Example 8

Travoprost formulations with alternative anionic surfactants. Batch size: 7.5 liters

| Ingredient | Quantity |
|---|---|
| travoprost | 0.300 g |
| boric acid | 30.75 g |
| trometamol | 4.95 g |
| | |
| surfactant: | |
| sodium lauryl ether sulfate 27% | 0.55 g |
| or lauryl sarcosinate sodium | 0.55 g |
| or lauryl ether sulfate triethanolamine | 0.55 g |
| | |
| manitol | 34.50 g |
| 2.0 M NaOH or diluted HCl | as required for final pH 5.8-6.2 |
| water for injection | for final volume of 7.5 l |

| Batch TVX 05 - Lauryl ether sulfat sodium | |
|---|---|
| Parameter | Time 0 |
| Appearance | Complies |
| pH | 6.09 |
| Travoprost assay (µg/ml) | 39,4 |
| Sum of all degradation products | < 0.05% |
| Microbiological control | < 10 CFU/100 ml |
| | |

| Batch TVX-06 - Lauryl sarcosinate sodium | |
|---|---|
| Parameter | Time 0 |
| Appearance | Complies |
| pH | 6.12 |
| Travoprost assay (µg/ml) | 40.18 |
| Sum of all degradation products | < 0.05% |
| Microbiological control | < 10 CFU/100 ml |

| Batch TVX-07 - Lauryl ether triethanolamine | |
|---|---|
| Parameter | Time 0 |
| Appearance | Complies |
| pH | 6.17 |
| Travoprost assay (µg/ml) | 39,42 |
| Sum of all degradation products | < 0.05% |
| Microbiological control | < 10 CFU/100 ml |

### Example 9

Comparative tests on the speed of dissolution of travoprost with different surfactants. Formulation for 7.5 liters:

| Ingredient | Quantity |
|---|---|
| travoprost | 0.300 g |
| boric acid | 30.75 g |
| trometamol | 4.95 g |
| | |
| surfactant: | |
| sodium lauryl ether sulfate 27% | 0.55 g |
| or Polysorbate 60 | 0.55 g |
| or Cremophor RH40 | 0.55 g |
| | |
| Manitol | 34.50 g |
| 3.0 M NaOH or diluted HCl | as required for final pH 5.8-6.2 |
| water for injection | for final volume of 7.5 l |

Temperature: 25°C
Method: as described in Example 6. All solutions were pre-equilibrated to 25°C.

The travoprost solution was made as follows. Travoprost, sodium lauryl ether sulfate and 20 ml of water were mixed in the amounts indicated in the above table in a glass vessel. The vessel was sonicated at room temperature until the components dissolved, which takes about 5 minutes. Then, 300 ml of water were added and sonicated for another 5 minutes. The temperature of the solution does not rise above 25°C.

| Batch | Time for dissolution of latanoprost |
|---|---|
| Batch TVX 017 with sodium lauryl ether sulphate | < 5 minutes |
| Batch TVX 18 with Cremophor RH40 | 16 minutes |
| Batch TVX 019 with Polysorbate 80 | 12 minutes |

### Example 10

A bimatoprost formulation of bach size 7.5 liters was prepared from the following components:

| Ingredient | Quantity |
|---|---|
| bimatoprost | 2.25 g |
| disodium phosphate | 34.9 g |
| monosodium phosphate monohydrate | 34.1 g |
| sodium lauryl ether sulfate (27%) | 0.55 g |
| propylene glycol | 8 ml |
| sodium chloride | 30.75 g |
| 1.0 M NaOH or diluted phosphoric acid | as required for final pH 6.6-6.8 |
| water for injection | for final volume of 7.5 l |

First, a bimatoprost solution was made as follows. Bimatoprost, sodium lauryl ether sulfate and propylene glycol were mixed in the amounts indicated in the above table in a glass vessel. The vessel was sonicated until the components dissolved, which takes about 10 minutes. Then, 15 ml of water were added and shaken gently for another 5 minutes.

In a separate vessel, the disodium phosphate, the monosodium phosphate monohydrate and the sodium chloride was dissolved in about 6 I of water (80% of the total volume of the batch size). When the solids were dissolved, the bimatoprost solution made as described in the preceding paragraph was added, followed by gentle shaking for 10 minutes. Then, the pH was set to be within the range of 6.6-6.8 with the 1.0 M sodium hydroxide solution or the diluted phosphoric acid as necessary. Next, water was added up to the final volume of 7.5 I.

The obtained mixture was filtered through a Millipore filter using a 0.2 µm membrane. The filtrate was dispensed into 2.5 ml PE bottles in a bottlepack system, and used in the tests of the following example 11.
A stability test was conducted on 3 separate batches of formulations. The formulations were stored at a temperature of 5-8°C for 30 days at a relative humidity of 60%.

| Batch BIX-01 | | |
|---|---|---|
| Parameter | Time 0 | Time 30 days |
| Appearance | Complies | Complies |
| pH | 6.71 | 6.79 |
| Bimatoprost assay (mg/ml) | 0.309 | 0.312 |
| Sum of all degradation products | < 0.05% | < 0.05% |
| Microbiological control | sterile | sterile |
| | | |

| Batch BIX-02 | | |
|---|---|---|
| Parameter | Time 0 | Time 30 days |
| Appearance | Complies | Complies |
| pH | 6.66 | 6.61 |
| Bimatoprost assay (mg/ml) | 0.296 | 0.293 |
| Sum of all degradation products | < 0.05% | < 0.05% |
| Microbiological control | sterile | Sterile |
| | | |

| Batch BIX-03 | | |
|---|---|---|
| Parameter | Time 0 | Time 30 days |
| Appearance | Complies | Complies |
| pH | 6.70 | 6.78 |
| Bimatoprost assay (mg/ml) | 0.305 | 0.306 |
| Sum of all degradation products | < 0.05% | < 0.05% |
| Microbiological control | sterile | Sterile |

The data from the above tables demonstrate that there was no noticeable loss of bimatoprost under the conditions used.

### Example 11

Bimatoprost formulations with alternative anionic surfactants. Batch size: 7.5 liters

| Ingredient | Quantity |
|---|---|
| bimatoprost | 2.25 g |
| disodium phosphate | 34.9 g |
| monosodium phosphate monohydrate | 34.1 g |
| | |
| surfactant: | |
| sodium lauryl ether sulfate 27% or | 0.55 g |
| or sodium lauryl sarcosinate or | 0.55 g |
| or lauryl ether sulfate triethanolamine propylene glycol | 0.55 g |
| sodium chloride | 8 ml |
| 1.0 M NaOH or diluted phosphoric acid | 30.75 g |
| | as required for final pH 6.6-6.8 |
| water for injection | |
| | for final volume of 7.5 I |

| Batch BIX 04 - Lauryl ether sulfat sodium | |
|---|---|
| Parameter | Time 0 |
| Appearance | Complies |
| pH | 6.70 |
| Bimatoprost assay (mg/ml) | 0.308 |
| Sum of all degradation products | <0.05% |
| Microbiological control | sterile |
| | |

| Batch BIX-05 - Lauryl sarcosinate sodium | |
|---|---|
| Parameter | Time 0 |
| Appearance | Complies |
| pH | 6.72 |
| Bimatoprost assay (mg/ml) | 0.288 |
| Sum of all degradation products | <0.05% |
| Microbiological control | sterile |

| Batch BIX-06 - Lauryl ether triethanolamine | |
|---|---|
| Parameter | Time 0 |
| Appearance | Complies |
| pH | 6.77 |
| Bimatoprost assay (mg/ml) | 0.298 |
| Sum of all degradation products | <0.05% |
| Microbiological control | sterile |

### Example 12

Bimatoprost formulations containing different concentrations of anionic surfactants. Formulation for 7.5 liters.
Anionic surfactant: Texapon N40
Temperature: room temperature not more than 30°C

| Batch | Time for dissolution of bimatoprost |
|---|---|
| Batch BIX 08 with 0.200 g surfactant | < 15 minutes |
| Batch BIX 09 with 0.400 g surfactant | < 15 minutes |
| Batch BIX 010 with 0.600 g surfactant | < 15 minutes |

With Chremophor or Polysorbate surfactants, bimatoprost does not dissolve without heating.

### Example 13

A travoprost/timolol formulation of bach size 7.5 litres was prepared from the following components:

| Ingredient | Quantity |
|---|---|
| travoprost | 0.300 g |
| timolol maleate | 47,25 g |
| boric acid | 30.75 g |
| trometamol | 4.95 g |
| sodium lauryl ether sulfate 27% | 0.55 g* |
| manitol | 34.50 g |
| 4.0 M NaOH or diluted HCl | as required for final pH 5.8-6.2 |
| water for injection | for final volume of 7.5 I |

| | |
|---|---|
| * of the 27%solution | |

First, a travoprost solution was made as follows. Travoprost, sodium lauryl ether sulfate and 20 ml of water were mixed in the amounts indicated in the above table in a glass vessel. The vessel was sonicated at room temperature until the components dissolved, which takes about 5 minutes. Then, 300 ml of water were added and sonicated for another 5 minutes. The temperature of the solution does not rise above 25°C.

In a separate vessel, the timolol maleate, the boric acid, the trometamol and the manitol was dissolved in about 6 I of water (80% of the total volume of the batch size). When the solids were dissolved, the travoprost solution made as described in the preceding paragraph was added, followed by gentle shaking for 10 minutes. Then, the pH was set to be within the range of 5.8-6.2 with the 1.0 M sodium hydroxide solution or the diluted hydrochloric acid as necessary. Next, water was added up to the final volume of 7.5 I.

The obtained mixture was filtered through a Millipore filter using a 0.2 µm membrane. The filtrate was dispensed into 2.5 ml PE bottles in a bottlepack system, and used in the tests of the following example 14.

### Example 14

The travoprost formulation of Example 13 was tested on its content of travoprost and degradation products of latanoprost:
- before and after the filtration through the 0.2 µm membrane of Example 13
- in a short stability test.
The results of 3 independent laboratory batches before and after filtration are as follows:

| Batch TVX-01 | | |
|---|---|---|
| Parameter | Before filtration | After filtration |
| Appearance | Complies | Complies |
| pH | 6.11 | 6.11 |
| Travoprost assay (µg/ml) | 41.2 | 41.3 |
| Timolol maleate (mg/ml) | 6.31 | 6.36 |
| Sum of all degradation products | < 0.05% | < 0.05% |
| Microbiological control | < 10 CFU/100 ml | sterile |
| | | |

| Batch TVX-02 | | |
|---|---|---|
| Parameter | Before filtration | After filtration |
| Appearance | Complies | Complies |
| pH | 6.07 | 5.92 |
| Travoprost assay (µg/ml) | 44.5 | 44.3 |
| Timolol maleate | 6.28 | 6.23 |
| Sum of all degradation products | < 0.05% | < 0.05% |
| Microbiological control | < 10 CFU/100 ml | sterile |
| | | |

| Batch TVX-03 | | |
|---|---|---|
| Parameter | Before filtration | After filtration |
| Appearance | Complies | Complies |
| pH | 6.09 | 6.09 |
| Travoprost assay (µg/ml) | 40.02 | 40.05 |
| Timolol assay | 6.51 | 6.55 |
| Sum of all degradation products | < 0.05% | < 0.05% |
| Microbiological control | < 10 CFU/100 ml | sterile |

The above data show that essentially no travoprost nor timolol was lost in the filtration step.

The stability test was conducted on 3 separate batches of formulations. The formulations were stored at a temperature of 25 +/- 2 °C for 30 days at a relative humidity of 60%. The data from the above tables demonstrate that there was no noticeable loss of travoprost under the conditions used.

| Batch TVX-01 | | |
|---|---|---|
| Parameter | Time 0 | 30 days |
| Appearance | Complies | Complies |
| pH | 6.11 | 6.05 |
| Travoprost assay (µg/ml) | 41.3 | 41.6 |
| Timolol maleate (mg/ml) | 6.36 | 6.37 |
| Sum of all degradation products | < 0.05% | < 0.05% |
| Microbiological control | sterile | sterile |
| | | |

| Batch TVX-02 | | |
|---|---|---|
| Parameter | Time 0 | 30 days |
| Appearance | Complies | Complies |
| pH | 5.92 | 5.99 |
| Travoprost assay (µg/ml) | 44.3 | 44.7 |
| Timolol maleate | 6.23 | 6.18 |
| Sum of all degradation products | < 0.05% | < 0.05% |
| Microbiological control | sterile | sterile |
| | | |

| Batch TVX-03 | | |
|---|---|---|
| Parameter | Time 0 | 30 days |
| Appearance | Complies | Complies |
| pH | 6.09 | 6.09 |
| Travoprost assay (µg/ml) | 40.05 | 40.12 |
| Timolol assay | 6.55 | 6.56 |
| Sum of all degradation products | < 0.05% | < 0.05% |
| Microbiological control | sterile | sterile |

## Claims

1. An aqueous ophthalmic composition comprising a prostaglandin and one or more anionic surfactants,
wherein the one or more anionic surfactant is selected from an alkali metal, C₁-C₃ alkylammonium, di(C₁-C₃ alkanol)ammonium, tri(C₁-C₃ alkanol)ammonium, or ammonium salts of:
a C₁₀-C₂₂alkyl sulfate,
a C₁₀-C₂₂ alkyl(oligooxyalkylene)sulfate, wherein the oligooxyalkylene moiety has from one to five oxy-C₁-C₆ alkylene moieties,
a C₄-C₂₂ alkyl sulfosuccinate ester,
a C₁₀-C₂₂ acylsarcosinate, or
a C₁₀-C₂₂ alkylcarboxylate;
and wherein
(A) the concentration of anionic surfactant in the ophthalmic composition is from 0.005 to 0.1 g/L, the concentration of the prostaglandin in the ophthalmic composition is from 0.001 to 0.1 g/L, and the ophthalmic composition contains less than 0.00005 g/L benzalkonium chloride;
or
(B) the concentration of anionic surfactant in the ophthalmic composition is from 0.01 to 0.045 g/L and the concentration of the prostaglandin in the ophthalmic composition is from 0.001 to 0.1 g/L.

2. The aqueous ophthalmic composition according to claim 1, wherein the prostaglandin is of the following formula (1): 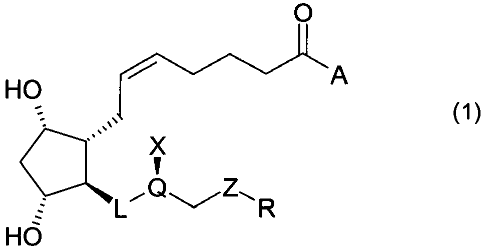 wherein
A is a linear or branched C₁-C₆ alkoxy moiety or a linear or branched mono- or di-C₁-C₆ alkylamino moiety;
L is a (CH₂)₂ moiety or a *trans*-CH=CH moiety;
Q is C, a CH moiety, or a CF moiety, with the proviso that when
(a) Q is C, X is O and the Q-X bond is a double bond,
(b) Q is CH, X is OH, and
(c) Q is CF, X is F;
R is a linear, branched or cyclic C₃-C₈ alkyl moiety, a phenyl moiety, or a phenyl moiety which is substituted with
(i) a linear or branched C₁-C₃ alkyl moiety,
(ii) a halogen, or
(iii) a linear or branched C₁-C₃ haloalkyl moiety; and
Z is O or a CH₂ moiety.

3. The aqueous ophthalmic composition according to claim 1 or 2, wherein the prostaglandin is a prostaglandin FP receptor agonist.

4. The aqueous ophthalmic composition according to any of claims 1 to 3, wherein the weight ratio of the prostaglandin to the anionic surfactant(s) in said composition is 1.0 or more.

5. The aqueous ophthalmic composition according to any of claims 1 to 4, wherein the amount of benzalkonium chloride or other cationic surfactants in the ophthalmic composition together is less than 0.00001 g/L in the composition.

6. The aqueous ophthalmic composition according to any of claims 1 to 5, wherein the oligooxyalkylene moieties of said alkyl(oligooxyalkylene)sulfate represent from one to five oxy-C₁-C₆ alkylene moieties, preferably oxyethylene moieties, linked via ether moieties.

7. The aqueous ophthalmic composition according to any of claims 1 to 5, wherein
(i) the C₁₀-C₂₂ alkyl sulfate is selected from sodium dodecylsulfate, potassium dodecylsulfate, triethanolammonium dodecylsulfate, diethanolammonium dodecylsulfate, ammonium dodecyl sulfate, sodium tetradecyl sulfate, triethanolammonium tetradecyl sulfates, sodium cocosulfates, sodium tallow alcohol sulfates, triethanolammonium C₁₂-C₁₄ alkylsulfates and sodium C₁₂-C₁₈ alkylsulfates;
(ii) the C₁₀-C₂₂ alkyl(oligooxyalkylene)sulfate is selected from sodium laureth sulfate, triethanolamine laureth sulfate, sodium trideceth sulfate, sodium myreth sulfate and sodium C₁₂-C₁₄ alkyl(oligooxyethylene) sulfates;
(iii) the C₄-C₂₂ alkyl sulfosuccinate ester is selected from disodium lauryl sulfosuccinate, sodium 1,4-bis(2-ethylhexyl)sulfosuccinate, sodium dioctylsulfosuccinate, sodium dicyclohexylsulfosuccinate and sodium 1,4-bis(1-methylheptyl)sulfosuccinate; and
(iv) the C₁₀-C₂₂ acylsarcosinate is selected from sodium lauroyl sarcosinate, potassium cocoyl hydrolysed collagen, triethanolamine cocoyl hydrolysed collagen, potassium undecenoyl hydrolysed collagen and triethanolamine abietoyl hydrolysed collagen.

8. The aqueous ophthalmic composition according to any of claims 1 to 7, wherein the prostaglandin is selected from latanoprost, bimatoprost, travoprost, tafluprost and unoprostone isopropyl.

9. The aqueous ophthalmic composition according to any of claims 1 to 8, wherein the ophthalmic composition additionally comprises a hydrophilic organic solvent, preferably a polyol, more preferably a C1-C6 alkylene glycol, a poly-C2-C6 alkylene glycol or a C1-C6 alkane triol.

10. The ophthalmic composition according to any one of claims 1 to 9, wherein the anionic surfactant is one or more selected from a lauryl ether sulfate such as sodium lauryl ether sulfate, or a lauroyl sarcosinate such as sodium lauroyl sarcosinate.

11. A method for manufacture of an aqueous ophthalmic composition according to any of claims 1 to 10, which comprises the following steps:
(i) dissolving a prostaglandin and one or more anionic surfactant in a hydrophilic organic solvent optionally in the presence of water, in water, or in an aqueous solvent; and
(ii) diluting the solution obtained in step (i) with water optionally including one or more ophthalmically acceptable excipients.

12. The method according to claim 11, wherein steps (i) and/or (ii) are performed at between 0 and 10°C, preferably at between 3 and 8°C.

13. The aqueous ophthalmic composition according to any one of claims 1 to 10 for use in a method of preventing or treating glaucoma or intraocular hypertension, or of providing neuroprotection to retinal tissues, by topical administration to an affected eye.

14. A packaged prostaglandin product, comprising the aqueous ophthalmic composition of any one of claims 1 to 10 sealed in a plastic container, wherein the plastic container is preferably a polyethylene or polypropylene container.

## Patentansprüche

1. Wässrige ophthalmische Zusammensetzung umfassend ein Prostaglandin und ein oder mehrere anionische Tenside,
worin das eine oder die mehreren anionischen Tenside ausgewählt ist/sind aus einem Alkalimetall-, C₁-C₃ Alkylammonium, Di(C₁-C₃ alkanol)ammonium, Tri(C₁-C₃ alkanol)ammonium oder Ammoniumsalz von:
einem C₁₀-C₂₂-Alkylsulfat,
einem C₁₀-C₂₂-Alkyl(oligooxyalkylen)sulfat, worin die Oligooxyalkylengruppe eine bis fünf Oxy-C₁-C₆-alkylengruppen aufweist,
einem C₄-C₂₂-Alkylsulfosuccinatester,
einem C₁₀-C₂₂-Acylsarcosinat oder
einem C₁₀-C₂₂-Alkylcarboxylat;
und worin
(A) die Konzentration an anionischem Tensid in der ophthalmischen Zusammensetzung von 0.005 bis 0.1 g/L beträgt, die Konzentration am Prostaglandin in der ophthalmischen Zusammensetzung von 0.001 bis 0.1 g/L beträgt und die ophthalmischen Zusammensetzung weniger als 0.00005 g/L Benzalkoniumchlorid enthält;
oder
(B) die Konzentration an anionischem Tensid in der ophthalmischen Zusammensetzung von 0.01 bis 0.045 g/L und die Konzentration am Prostaglandin in der ophthalmischen Zusammensetzung von 0.001 bis 0.1 g/L beträgt.

2. Die wässrige ophthalmische Zusammensetzung gemäß Anspruch 1, worin das Prostaglandin die folgende Formel (1) aufweist: 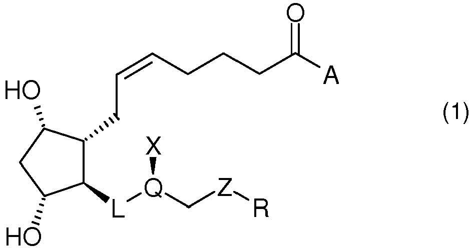 worin
A eine lineare oder verzweigte C₁-C₆ Alkoxygruppe oder eine lineare oder verzweigte Mono- oder Di-C₁-C₆-alkylaminogruppe ist;
L eine (CH₂)₂-Gruppe oder eine *trans*-CH=CH-Gruppe ist;
Q C, eine CH-Gruppe oder eine CF-Gruppe ist, mit der Bedingung, dass wenn
(a) Q C ist, X O ist und die Q-X - Bindung eine Doppelbindung ist,
(b) Q CH ist, X OH ist und
(c) Q CF ist, X F ist;
R eine lineare, verzweigte oder cyclische C₃-C₈ Alkylgruppe, eine Phenylgruppe oder eine Phenylgruppe ist, die substituiert ist mit
(i) einer linearen oder verzweigten C₁-C₃ Alkylgruppe,
(ii) einem Halogen oder
(iii) einer linearen oder verzweigten C₁-C₃ Haloalkylgruppe; und
Z O oder eine CH₂-Gruppe ist.

3. Die wässrige ophthalmische Zusammensetzung gemäß Anspruch 1 oder 2, worin das Prostaglandin ein Prostaglandin-FP-Rezeptor-Agonist ist.

4. Die wässrige ophthalmische Zusammensetzung gemäß einem der Ansprüche 1 bis 3, worin das Gewichtsverhältnis des Prostaglandins zum anionischen Tensid oder den anionischen Tensiden in der Zusammensetzung 1,0 oder mehr beträgt.

5. Die wässrige ophthalmische Zusammensetzung gemäß einem der Ansprüche 1 bis 4, worin die Menge an Benzalkoniumchlorid oder anderen kationischen Tensiden in der ophthalmische Zusammensetzung zusammen weniger als 0.00001 g/L beträgt.

6. Die wässrige ophthalmische Zusammensetzung gemäß einem der Ansprüche 1 bis 5, worin die Oligooxyalkylengruppen des Alkyl(oligooxyalkylen)sulfats ein bis fünf Oxy-C₁-C₆-alkylengruppen, vorzugsweise Oxyethylengruppen, sind, die über Ethergruppen verknüpft sind.

7. Die wässrige ophthalmische Zusammensetzung gemäß einem der Ansprüche 1 bis 5, worin
(i) das C₁₀-C₂₂Alkylsulfat ausgewählt ist aus Natriumdodecylsulfat, Kaliumdodecylsulfat, Triethanolammoniumdodecylsulfat, Diethanolammoniumdodecylsulfat, Ammoniumdodecylsulfat, Natriumtetradecylsulfat, Triethanolammoniumtetradecylsulfaten, Natriumcocoylsulfaten, Natrium-Talgalkoholsulfate, Triethanolammonium-C₁₂-C₁₄ -alkylsulfate und Natrium-C₁₂-C₁₈-alkylsulfate;
(ii) das C₁₀-C₂₂ -Alkyl(oligooxyalkylen)sulfat ausgewählt ist aus Natriumlaurethsulfat, Triethanolaminlaurethsulfat, Natriumtridecethsulfat, Natriummyrethsulfate und Natrium-C₁₂-C₁₄-alkyl(oligooxyethylen)sulfaten;
(iii) der C₄-C₂₂ Alkylsulfosuccinatester ausgewählt ist aus Dinatriumlaurylsulfosuccinat, Natrium-1,4-bis(2-ethylhexyl)sulfosuccinat, Natriumdioctylsulfosuccinate, Natriumdicyclohexylsulfosuccinat und Natrium-1,4-bis(1-methylheptyl)sulfosuccinat; und
(iv) das C₁₀-C₂₂ Acylsarcosinat ausgewählt ist aus Natriumlauroylsarcosinat, Kaliumcocoyl-hydrolysiertes Kollagen, Triethanolamin-cocoyl-hydrolysiertes Kollagen, Kaliumundecenoyl-hydrolysiertes Kollagen und Triethanolaminabietoyl-hydrolysiertes Kollagen.

8. Die wässrige ophthalmische Zusammensetzung gemäß einem der Ansprüche 1 bis 7, worin das Prostaglandin ausgewählt ist aus Latanoprost, Bimatoprost, Travoprost, Tafluprost und Unoprostonisopropyl.

9. Die wässrige ophthalmische Zusammensetzung gemäß einem der Ansprüche 1 bis 8, worin die ophthalmische Zusammensetzung ferner ein hydrophiles organisches Lösungsmittel enthält, vorzugsweise ein Polyol, bevorzugter ein C₁-C₆ Alkylenglycol, ein Poly-C₂-C₆ Alkylenglycol oder ein C₁-C₆ Alkantriol.

10. Die wässrige ophthalmische Zusammensetzung gemäß einem der Ansprüche 1 bis 9, worin das anionische Tensid eines oder mehr ausgewählt aus Laurylethersulfat wie Natriumlaurylethersulfat oder ein Lauroylsarcosinat wie Natriumlauroylsarcosinat ist.

11. Verfahren zur Herstellung einer wässrigen ophthalmische Zusammensetzung gemäß einem der Ansprüche 1 bis 10, das die folgenden Schritte umfasst:
(i) Lösen eines Prostaglandins und einem oder mehrerer anionische Tenside in einem hydrophilen organischen Lösungsmittel, wahlweise in Gegenwart von Wasser, in Wasser oder in einem wässrigen Lösungsmittel; und
(ii) Verdünnen der in Schritt (i) erhaltenen Lösung mit Wasser, das wahlweise einen oder mehrere ophthalmisch akzeptable Hilfsstoffe enthält.

12. Das Verfahren gemäß Anspruch 11, worin Schritte (i) und/oder (ii) zwischen 0 und 10°C, vorzugsweise zwischen 3 und 8°C durchgeführt werden.

13. Die wässrige ophthalmische Zusammensetzung gemäß einem der Ansprüche 1 bis 10 zur Verwendung in einem Verfahren zur Vorbeugung gegen oder zur Behandlung von Glaukom oder erhöhtem Innenaugendruck, oder zur Verleihung von Neuroprotektion für retinale Gewebe, durch topische Anwendung auf ein betroffenes Auge.

14. Verpacktes Prostaglandinprodukt, umfassend die wässrige ophthalmische Zusammensetzung gemäß einem der Ansprüche 1 bis 10 verschlossen in einem Plastikbehälter, wobei der Plastikbehälter vorzugsweise ein Polyethylen- oder Polypropylenbehälter ist.

## Revendications

1. Composition aqueuse à usage ophtalmique comprenant une prostaglandine et un ou plusieurs tensioactif(s) anionique(s),
dans laquelle le ou les tensioactif(s) anionique(s) est/sont choisi(s) parmi un métal alcalin, un alkylammonium en C₁ à C₃, un di (alcanol en C₁ à C₃) ammonium, un tri (alcanol en C₁ à C₃) ammonium, et les sels d'ammonium de :
un alkylsulfate en C₁₀ à C₂₂,
un alkyl(oligoxyalkylène)sulfate en C₁₀ à C₂₂ dont le fragment oligoxyalkylène possède de un à cinq fragments oxyalkylène en C₁ à C₆,
un ester alkylsulfosuccinate en C₄ à C₂₂,
un acylsarcosinate en C₁₀ à C₂₂, ou
un alkylcarboxylate en C₁₀ à C₂₂ ;
et dans laquelle
(A) la concentration de tensioactif anionique dans la composition à usage ophtalmique est de 0,005 à 0,1 g/l, la concentration de la prostaglandine dans la composition à usage ophtalmique est de 0,001 à 0,1 g/l, et la composition à usage ophtalmique contient moins de 0,00005 g/l de chlorure de benzalkonium ;
ou
(B) la concentration de tensioactif anionique dans la composition à usage ophtalmique est de 0,01 à 0,045 g/l et la concentration de la prostaglandine dans la composition à usage ophtalmique est de 0,001 à 0,1 g/l.

2. Composition aqueuse à usage ophtalmique selon la revendication 1, dans laquelle la prostaglandine répond à la formule (1) suivante : 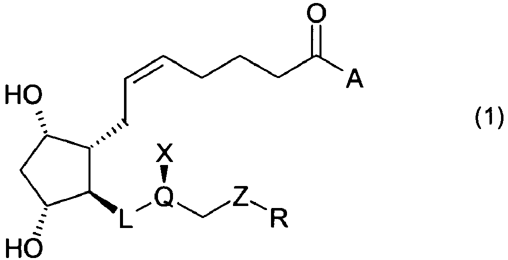 dans laquelle
A est un fragment alcoxy en C₁ à C₆ linéaire ou ramifié ou un fragment mono- ou di- (alkyle en C₁ à C₆)amino linéaire ou ramifié ;
L est un fragment (CH₂)₂ ou un fragment trans-CH=CH ;
Q est C, un fragment CH ou un fragment CF, avec les conditions suivantes :
(a) quand Q est C, X est O et la liaison Q-X est une double liaison,
(b) quand Q est CH, X est OH, et
(c) quand Q est CF, X est F ;
R est un fragment alkyle en C₃ à C₈ linéaire, ramifié ou cyclique, un fragment phényle, ou un fragment phényle qui est substitué par
(i) un fragment alkyle en C₁ à C₃ linéaire ou ramifié,
(ii) un halogène, ou
(iii)un fragment halogénoalkyle en C₁ à C₃ linéaire ou ramifié ; et
Z est O ou un fragment CH₂.

3. Composition aqueuse à usage ophtalmique selon la revendication 1 ou 2, dans laquelle la prostaglandine est un agoniste du récepteur FP de la prostaglandine.

4. Composition aqueuse à usage ophtalmique selon l'une quelconque des revendications 1 à 3, dans laquelle le rapport en poids de la prostaglandine sur le ou les tensioactif(s) anionique(s) dans ladite composition est de 1,0 ou plus.

5. Composition aqueuse à usage ophtalmique selon l'une quelconque des revendications 1 à 4, dans laquelle la quantité de chlorure de benzalkonium ou d'autres tensioactifs cationiques, pris ensemble, dans la composition à usage ophtalmique, est inférieure à 0,00001 g/l dans la composition.

6. Composition aqueuse à usage ophtalmique selon l'une quelconque des revendications 1 à 5, dans laquelle les fragments oligoxyalkylène dudit alkyl(oligoxyalkylène)sulfate représentent de un à cinq fragments oxyalkylène en C₁ à C₆, de préférence des fragments oxyéthylène, liés via des fragments éther.

7. Composition aqueuse à usage ophtalmique selon l'une quelconque des revendications 1 à 5, dans laquelle
(i) l'alkylsulfate en C₁₀ à C₂₂ est choisi parmi le dodécylsulfate de sodium, le dodécylsulfate de potassium, le dodécylsulfate de triéthanolammonium, le dodécylsulfate de diéthanolammonium, le dodécylsulfate d'ammonium, le tétradécylsulfate de sodium, les tétradécylsulfates de triéthanolammonium, les (coprah-yl)sulfates de sodium, les (suif-yl)alcool-sulfates de sodium, les alkylsulfates en C₁₂ à C₁₄ de triéthanolammonium, et les alkylsulfates de sodium en C₁₂ à C₁₈ ;
(ii) le (alkyle en C₁₀ à C₂₂)- (oligoxyalkylène) sulfate est choisi parmi le laureth-sulfate de sodium, le laureth-sulfate de triéthanolamine, le tridéceth-sulfate de sodium, le mureth-sulfate de sodium et les (alkyle en C₁₂ à C₁₄)-(oligoxyalkylène) sulfates de sodium ;
(iii)l'ester alkylsulfosuccinate en C₄ à C₂₂ est choisi parmi le laurylsulfosuccinate disodique, le 1,4-bis(2-éthylhexyl)sulfosuccinate de sodium, le dioctylsulfosuccinate de sodium, le dicyclohexylsulfosuccinate de sodium et le 1,4-bis(1-méthylheptyl)sulfosuccinate de sodium ; et
(iv) l'alkylsarcosinate en C₁₀ à C₂₂ est choisi parmi le lauroylsarcosinate de sodium, le (coprah-yl)collagène hydrolysé potassique, le (coprah-yl)collagène hydrolysé de triéthanolamine, l'undécénoyl-collagène hydrolysé potassique, et l'abiétoyl-collagène hydrolysé de triéthanolamine.

8. Composition aqueuse à usage ophtalmique selon l'une quelconque des revendications 1 à 7, dans laquelle la prostaglandine est choisie parmi le latanoprost, le bimatoprost, le travoprost, le tafluprost et l'unoprostone isopropyle.

9. Composition aqueuse à usage ophtalmique selon l'une quelconque des revendications 1 à 8, laquelle composition à usage ophtalmique comprend de plus un solvant organique hydrophile, de préférence un polyol, plus préférablement un alkylèneglycol en C₁ à C₆, un poly(alkylèneglycol en C₂ à C₆) ou un alcanetriol en C₁ à C₆.

10. Composition aqueuse à usage ophtalmique selon l'une quelconque des revendications 1 à 9, dans laquelle le tensioactif anionique est un ou plusieurs choisi(s) parmi un lauryléthersulfate tel que le lauryléthersulfate de sodium, et un lauroylsarcosinate tel que le lauroylsarcosinate de sodium.

11. Procédé de fabrication d'une composition aqueuse à usage ophtalmique selon l'une quelconque des revendications 1 à 10, qui comprend les étapes suivantes :
(i) dissolution d'une prostaglandine et d'un ou plusieurs tensioactif(s) anionique(s) dans un solvant organique hydrophile éventuellement en présence d'eau, dans de l'eau, ou dans un solvant aqueux ; et
(ii) dilution de la solution obtenue à l'étape (i) avec de l'eau contenant éventuellement un ou plusieurs excipient(s) acceptable(s) du point de vue ophtalmique.

12. Procédé selon la revendication 11, dans lequel les étapes (i) et/ou (ii) sont effectuées entre 0 et 10°C, de préférence entre 3 et 8°C.

13. Composition aqueuse à usage ophtalmique selon l'une quelconque des revendications 1 à 10 pour utilisation dans une méthode de prévention ou de traitement d'un glaucome ou d'une hypertension intraoculaire, ou pour conférer une neuroprotection aux tissus rétiniens, par administration topique à un oeil affecté.

14. Produit à base de prostaglandine conditionné, comprenant la composition aqueuse à usage ophtalmique de l'une quelconque des revendications 1 à 10 scellée dans un récipient plastique, dans lequel le récipient plastique est de préférence un récipient en polyéthylène ou en polypropylène.
